# EUROPEAN PATENT APPLICATION

(11) **EP 1 302 211 A1**
(43) Date of publication of application: **16.04.2003**
(21) Application number: 01124484.5
(22) Date of filing: 12.10.2001
(51) Int. Cl.: A61M 5/32

(54) **Multipurpose inoculating needle and method for its manufacture**

(71) Applicant: Catarsi Ing. Piero & C. S.r.l., 56012 Calcinaia (IT)
(72) Inventor: Pardini, Mario, 56019 Vecchiano (Pisa) (IT); Santerini, Sante, 56021 Cascina (Pisa) (IT); Catarsi, Piero, 56012 Calcinaia (Pisa) (IT); Machhi, Romualdo, 56100 Pisa (IT)
(74) Representative: Bardini, Marco Luigi

(57) **Abstract**

The inoculator needle - for phlebology, for intramuscular and intravenous use, and hence multipurpose - with penetrating lance and axial hole, has four lateral holes (3, 5) and an occlusion (F7) of the axial hole between said lateral holes and the penetrating lance (1A).

## Description

The present invention relates to a multipurpose inoculating needle and to the method for its manufacture. The common needle used for intramuscular or intravenous injections has the following dimensional characteristics: hole of 0.7 mm with length of 30 mm (for a syringe of up to 2.5 cc) and hole of 0.8 mm with length of 40 mm (for a syringe of 5 to 10 cc). Instead, the needle used in phlebology has totally different dimensions in terms of gauge and length. This inoculator has the following dimensions: hole of 0.3 mm for a length of 18 mm or (less widely used) hole of 0.4 mm for a length of 25 mm.

In daily medical practice, phlebology specialists, angiology specialists, vascular surgeons, dermatologists and oftentimes some general practitioners frequently need to inoculate into veins (small and very small veins) specific drugs, all deriving from the aliphatic series, particularly active on phlogistic tissues, which induce the sclerotisation and hence the occlusion of the treated vessel; only in this way will the specialist be able to achieve significant and often definitive therapeutic results in certain specific pathologies.

The phlebologic inoculator with a hole diameter of 0.30 mm and a length of 18 mm dispenses the drug into the vessel lumen from a single hole, the one obtained - as in all needles, also for non specialist use - in the terminal part and specifically in the flute beak penetrating lance.

This phlebologic inoculator has a specific limitation which has always reduced the therapeutic effectiveness provided by specialist physicians: the single outlet hole causes a very poor distribution of the drug with sclerotising action into the vessel lumen, and in particular the semi-circumference of the vein in direct contact with the hole of the penetrating lance is abundantly sprayed, whilst a small or certainly lesser quantity of drug comes in contact with the opposite semi-circumference of the vessel, also because it finds an additional hindrance caused by what can be defined as the "roof" of the penetrating lance, in which the single outlet hole forms a jet that is more lateral than axial. The therapeutic results thereby derived, therefore, are often aleatory, since the part of the vessel wall that is sprayed less abundantly will be subjected to a lesser sclerotising action, thus making it possible to have a more or less rapid re-canalisation, which robs the therapy of all its effectiveness.

A quite remote solution provided a pervious needle with two opposite holes immediately upstream of the lance, to attempt to overcome the drawback of the poor intra-vessel distribution of the sclerotising liquid; this simple solution does entail some advantages in terms of the homogeneity of distribution of the drug on the vessel walls, but only partly solves the problem of the correct spraying, i.e. of the uniformity of distribution of the drug between needle and vein.

Problems similar to those briefly described above are also encountered with intravenous needles; such problems are due to the single axial dispensing and to the presence of a hollow lance.

Problems araising from an undispersed dispensing of the injected liquid are also encountered with needles for intramuscular injections, which require a quick and spread absorption of the injected liquid by the tissue surrounding the lance end of the needle. The present invention solves the problems described above in a manner that is satisfactory in all aspects, both productive and functional, as shall become readily apparent from the description that follows.

A first subject of the invention is a multipurpose inoculator needle - i.e. one that is both phlebologic, and for intramuscular and intravenous use - with penetrating lance and axial hole, which has: at least two lateral holes between the axial hole and the outside; and an occlusion of the axial hole between said lateral holes and the lumen of the penetrating lance.

The axes of the lateral holes can preferably be orthogonal to the longitudinal geometric axis of the needle.

Two lateral holes can be coaxial or with mutually angled axes.

Advantageously, said needle can have at least four lateral holes, upstream of the occlusion. In particular, two pairs of coaxial lateral holes can be provided, the axis of the holes of a pair lying in an axial plane orthogonal to the axial plane in which lies the axis of the holes of the other pair of holes.

A second subject of the invention is a method for the manufacture of an inoculator needle of the kind defined above, comprising: an operation of boring the wall of the needle, to obtain lateral holes; and an operation of occluding the axial hole between said lateral hole and the penetrating end of the needle.

The occlusion can be obtained:
- by pinching and deforming the wall of the needle cannula;
- or by introducing occluding material into the axial hole in correspondence with the penetrating lance;
- or yet again by flattening the end of the needle cannula, and by subsequently machining the flattened area to form the penetrating lance or tip;
- or yet again by rolling the end of the cannula, whereby the penetrating tip is also obtained.

The lateral holes can be obtained by electron discharge machining or other methods that do not generate residual shavings or in which all metal residues can be safely removed.

The invention shall be better understood following the description and the accompanying drawing, which shows a practical, non limiting embodiments of the invention. In the drawing:
Figures 1 and 2 show a first embodiment, in a partially sectioned lateral view and in the section according to II-II of Figure 1;
Figure 3 shows a variation, in a view similar to that of Figure 1;
Figures 4, 5 and 6 show a second embodiment, in a lateral view of an intermediate machining phase, in the view from the line V-V and in the lateral view after a final mechanical machining, and
Figures 7 and 8 show two additional embodiments.

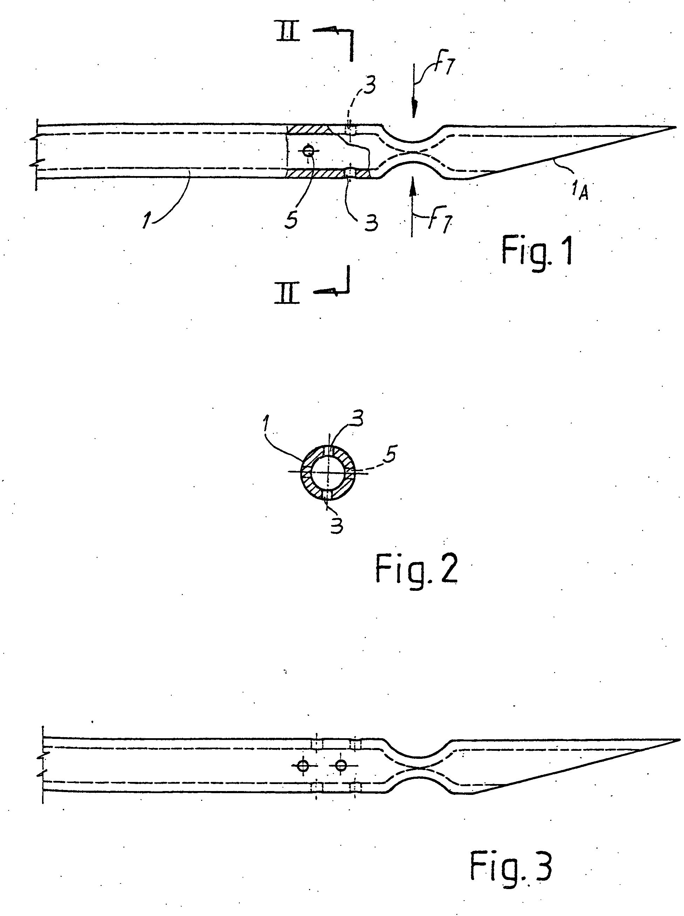

According to Figures 1 and 2, at a limited distance from the pervious perforating lance 1A of a tubular needle 1, two pairs of coaxial holes 3 and 5 are formed, the axes of said holes being orthogonal to the axis of needle 1 and lying in mutually orthogonal axial planes. Between end 1A and holes 3, an occlusion of the cannula of needle 1 is provided, obtained by a deformation, in practice a pinching 7 of tubular wall 7, by action according to radial directions, as indicated by arrows f7 of Figure 1.

The variation of Figure 3 comprises four pairs of lateral holes, which can be formed with axes parallel in pairs or all at mutually offset angles.

In all cases, the holes of each pair are offset to a limited extent from those of the other pair or pairs, to limit the weakening due to the holes.

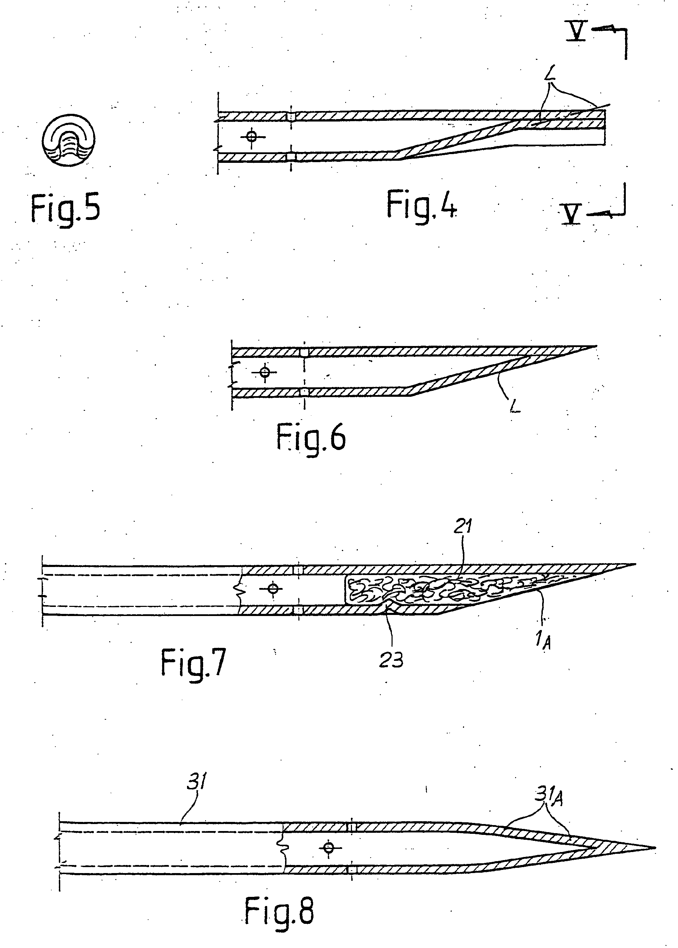

In the embodiments of Figures 4 through 6, penetrating lance 11A is obtained by preliminarily causing, at the end of the needle, a progressive, flattening, possibly modelled, of the tubular wall of the needle, thereby creating the occlusion; in a subsequent machining operation to remove the material of the flattened wall, the penetrating lance is obtained by removal along line L.

In the embodiment of Figure 7, the occlusion of penetrating lance 1A is obtained without deforming the tubular wall, but rather by applying an occluding material 21 into the lance obtained in the shape of a flute beak. At least a slight undercut 23 can be provided, to assure the securing of said occluding material 21.

Figure 8 shows the closure by rolling of end 31A of the cannula 31, whereby the formation of penetration tip 31A is also obtained.

With the solutions described above a good multipurpose inoculator needle is obtained, which guarantees the therapeutic result - also prolonged over time - because it allows to distribute the injected drug in a uniform manner into the tissue that undergoes the inoculation or onto the entire vessel wall of the vein into which the needle has penetrated. The closure of the exit hole in the penetrating lance is obtained, leaving the terminal lance with the sole task of penetration. Moreover, forming at least four holes with non coplanar axes, of calculated and appropriate diameter, immediately upstream of the lance, a regular distribution of injected or inoculated liquid is practically obtained over the whole area of the tissue or of the wall of the vessel thrust back by the needle. A considerable pressure of the liquid in the carrying channel can be exploited, thereby obtaining, from the lateral holes, jets that strike the vessel walls with considerable force and which cause such a vortical motion as to guarantee a perfect spraying of the whole surface of the walls of the vein around the needle penetrated therein or in all the tissue penetrated by the needle.

Thanks to the fact that the holes are not coplanar, the penetrating action is made easier and, in addition, the possible mechanical weakening of the inoculator needle is reduced.

A standard inoculator of 0.30 x 18 mm with an axial hole of 0.17 mm can be manufactured, obtaining four not coplanar holes with a diameter of 0.09 mm, thereby maintaining a good outlet pressure from each of the holes. Proportional dimensions are adopted for needles destined to hypodermic, intramuscular or other purposes.

In a practical test, the needle thereby obtained yielded excellent results both in terms of jet pressure and of the good resistance of the stainless steel 300, material used for the manufacture of all inoculators, including the 30 x 18 mm phlebologic one.

It is understood that the drawing shows but an example, provided solely as a practical demonstration of the invention, since said invention may vary in its forms and dispositions without thereby departing from the scope of the concept informing the invention. The presence of any reference numbers in the accompanying claims has the purpose of facilitating the reading of the claims - with reference to the description and to the drawing - and does not limit the scope of protection represented by the claims. For instance, single lateral holes may be obtained instead of pairs of coaxial holes, multiple single holes being positioned at different levels along the development of the lance end area of the needle.

## Claims

1. An inoculator needle (1) with penetrating lance (1A) and axial hole, **characterised in that** it is provided with at least two lateral holes (3) between the axial hole and the outside and with an occlusion (F7) of the axial hole between said lateral holes and the penetrating lance.

2. An inoculator needle (1), for phlebologic, intramuscular, intravenous or other uses, with penetrating lance (1A) and axial hole, **characterised in that** it is provided with at least four lateral holes (3, 5) between the axial hole and the outside and with an occlusion (F7) of the axial hole between said lateral holes and the penetrating lance.

3. An inoculator needle as claimed in claim 1 or 2, **characterised in that** two lateral holes (3, 5) are coaxial and opposite.

4. An inoculator needle as claimed at least in claim 2 or 3, **characterised in that** it has two pairs of coaxial lateral holes, the axis of the holes of one pair (3) lying in an axial plane orthogonal to the axial plane in which lies the axis of the holes of the other pair of holes (5).

5. A method for obtaining an inoculator needle (1) as claimed in at least one of the previous claims, **characterised in that** it comprises: operations of boring the needle wall, to obtain lateral holes (3, 5); and an operation of occluding (F7) the axial hole between said lateral holes and the penetrating end of the needle.

6. A method for manufacturing an inoculator needle (1) as claimed in claim 5, **characterised in that** the occlusion (F7) is obtained by pinching or otherwise deforming the wall of the needle cannula.

7. A method for manufacturing an inoculator needle (1) as claimed in claim 5, **characterised in that** the occlusion (F7) is obtained by introducing occluding material into the axial hole in correspondence with the penetrating lance (1A).

8. A method for manufacturing an inoculator needle (1) as claimed in claim 5, **characterised in that** the occlusion (F7) is obtained by flattening the end of the needle cannula, and by subsequently removing material from the flattened area to form the penetrating lance tip (1A).

9. A method for manufacturing an inoculator needle (1) as claimed at least in claim 5, **characterised in that** the occlusion (F7) is obtained by rolling the end of the cannula, whereby also the penetrating tip (1A) is obtained.

10. A method as claimed in claim 5, **characterised in that** the holes (3, 5) are obtained by electron discharge machining.
